# EUROPEAN PATENT APPLICATION

(11) **EP 4 843 581 A1**
(43) Date of publication of application: **25.11.2026**
(21) Application number: 25305715.2
(22) Date of filing: 20.05.2025
(51) Int. Cl.: A61K 38/00, C07K 14/005

(54) **INFLUENZA VIRUS POLYMERASE INHIBITORY PEPTIDE**

(71) Applicant: Centre National de la Recherche Scientifique, 75016 Paris (FR); Université Grenoble Alpes, 38400 Saint-Martin-d'Hères (FR); Institut Pasteur, 75015 Paris (FR); Institut National de la Santé et de la Recherche Médicale, 75013 Paris (FR); Commissariat à l'Energie Atomique et aux Energies Alternatives, 75015 Paris (FR)
(72) Inventor: HART, Darren, 38360 Noyarey (FR); FLOREZ PRADA, Alberto, 38000 Grenoble (FR); BURMEISTER, Wilhelm, 38000 Grenoble (FR); ISEL-GRIFFITHS, Catherine, 75006 Paris (FR); NAFFAKH, Nadia, 92240 Malakoff (FR)
(74) Representative: Novagraaf Technologies

(57) **Abstract**

The present invention relates to an influenza virus polymerase inhibitory peptide, comprising an amino acid sequence chosen among X₁DX₃NPX₆LLFLX₁₁X₁₂PX₁₄Q (SEQ ID NO: 1), DX₃NPX₆LLFLX₁₁X₁₂PX₁₄Q (SEQ ID NO: 2), X₁DX₃NPX₆LLFLX₁₁X₁₂PX₁₄ (SEQ ID NO: 3), and DX₃NPX₆LLFLX₁₁X₁₂PX₁₄ (SEQ ID NO: 4), wherein:
- X₁, when present, is chosen from V, I, L, M, Y, Q and W,
- X₃ is chosen from Y, F, I and M,
- X₆ is chosen from W and Y,
- X₁₁ is chosen from R, Q, L, K and V,
- X₁₂ is chosen from K, L, P, Q, V, F, and M, and
- X₁₄ is chosen from K, A, R Q, K, V, S and N.

The present invention also relates to a nucleic acid encoding the peptide, an expression vector into which the nucleic acid according is inserted, a cell transfected with the expression vector, a vector for delivery of the nucleic acid, a medicament comprising the peptide, or the nucleic acid, or the vector.

The present invention relates in addition to the peptide, or the nucleic acid, or the vector, for use as a medicament, especially for use in the treatment of influenza.

## Description

### Technical field

The present invention refers to influenza virus polymerase inhibitory peptides, vectors for delivery of nucleic acids encoding the peptides, and their use as a medicament.

Therefore, the present invention has utility in medical field, especially in the field of influenza therapy.

In the description below, the references into brackets ([ ]) refer to the listing of references situated at the end of the text.

### Background of the Invention

Influenza virus persists as a significant global health challenge, remaining the most prevalent viral respiratory disease despite longstanding vaccination efforts. Its high mutational capacity and zoonotic potential continuously threaten the emergence of new pandemic strains. The limited efficacy of current antiviral drugs against rapidly evolving variants showcases the urgent need for novel therapeutic strategies with innovative mechanisms of action and delivery methods.

Previous findings showed that a 15 amino acid fragment from the N-terminal region of PB1, a subunit of the influenza polymerase (FluPol), could reduce viral replication by disrupting the PA-PB1 interaction, which is crucial for FluPol assembly and viral replication. Even if peptide-based antiviral therapeutics offer a promising avenue due to their specificity and ability to disrupt critical protein-protein interactions essential for viral replication, unmodified peptides face substantial clinical challenges, including short plasma half-life, low affinity and selectivity, and poor cellular uptake owing to low membrane permeability. Commonly, peptides have been chemically modified or tagged with cell-penetrating motifs to enhance membrane permeability, but success has been limited for linear peptides.

Thus, a need exists of alternative peptides to enhance antiviral potential. The present invention fulfills these and other needs.

### Description of the invention

Thanks to extensive research work in which hundreds of millions of randomized peptide variants were screened, the inventors have designed high affinity peptides able to reduce viral replication and to enhance antiviral potential compared to the wild-type peptide (MDVNPTLLFLKVPAQ (SEQ ID NO: 5)) from which the peptides of the invention are derived (this wild-type peptide being disclaimed from the scope of the invention).

Indeed, surprisingly, the results of their work demonstrated that these peptides significantly disrupted the PA-PB1 interaction in both influenza A and B and reduced FluPol activity more effectively than the wild-type peptide.

Furthermore, the inventors have found surprisingly that utilizing mRNA lipid nanoparticle (LNP) technology for intracellular delivery, effective inhibition of viral replication in vitro occurred without observable toxicity.

All together, this approach not only overcomes historical barriers associated with peptide therapeutics including efficacy and vectorization but also provides a novel framework for antiviral drug development.

Accordingly, in a first aspect, the present invention provides an influenza virus polymerase inhibitory peptide, comprising an amino acid sequence chosen among X₁DX₃NPX₆LLFLX₁₁X₁₂PX₁₄Q (SEQ ID NO: 1), OX₃NPX₆LLFLX₁₁X₁₂PX₁₄Q (SEQ ID NO: 2), X₁DX₃NPX₆LLFLX₁₁X₁₂PX₁₄ (SEQ ID NO: 3), and DX₃NPX₆LLFLX₁₁X₁₂PX₁₄ (SEQ ID NO: 4), wherein:
- X₁, when present, is an amino acid chosen from V, I, L, M, Y, Q and W,
- X₃ is an amino acid chosen from Y, F, I and M,
- X₆ is an amino acid chosen from W and Y,
- X₁₁ is an amino acid chosen from R, Q, L, K and V,
- X₁₂ is an amino acid chosen from K, L, P, Q, V, F, and M, and
- X₁₄ is an amino acid chosen from K, A, R Q, K, V, S and N.

"Influenza virus polymerase inhibitory peptide" refers herein to a peptide that totally or partially inhibits influenza A, B, C and/or D virus replication, preferably influenza A. Inhibition may be at least 50%, or at least 60%, or at least 70%, or at least 80%, or at least 90%, or even 100% inhibition. Advantageously, the peptide of the invention may competitively inhibit protein-protein interaction of the PA and PB1 subunits of the heterotrimeric viral RNA polymerase complex, and thereby cause inhibition of virus replication, and antiviral activity. Percentage of inhibition may be quantified by any technique known in the state of the art, such as Biolayer Interferometry (BLI), Protein Complementation Assay (PCA) sur as Luciferase Complementation Assay, Co-immunoprecipitation, Surface Plasmon Resonance (SPR), Fluorescence Resonance Energy Transfer (FRET) or Fluorescence Polarization (FP), this list being non exhaustive.

The peptide is an isolated peptide, i.e. a peptide that has been separated from its natural biological context or source, typically chemically synthesized or purified from a complex mixture, such as tissue or cell extracts, or expressed using recombinant technologies as a small fragment of the larger PB1 protein.

Advantageously, the peptide of the invention competitively inhibits protein-protein interaction of the PA and PB1 subunits of the heterotrimeric viral RNA polymerase complex of at least one among influenza virus types A and B.

According to the invention, when X₁ is absent, the peptide comprises the amino acid sequence DX₃NPX₆LLFLX₁₁X₁₂PX₁₄Q (SEQ ID NO: 2).

In an embodiment of the invention, the peptide comprises the sequence chosen among X₁DX₃NPX₆LLFLX₁₁X₁₂PX₁₄Q (SEQ ID NO: 1) and X₁DX₃NPX₆LLFLX₁₁X₁₂PX₁₄ (SEQ ID NO: 3), wherein:
- X₁ is chosen from V, I, L and W,
- X₃ is chosen from Y, F and M,
- X₆ is chosen from W and Y,
- X₁₁ is chosen from R, K and L,
- X₁₂ is chosen from K and Q, and
- X₁₄ is chosen from K and A.

In another embodiment of the invention, the peptide comprises the sequence chosen among X₁DX₃NPX₆LLFLX₁₁X₁₂PX₁₄Q (SEQ ID NO: 1) and X₁DX₃NPX₆LLFLX₁₁X₁₂PX₁₄ (SEQ ID NO: 3), wherein:
- X₁ is chosen from V and I,
- X₃ is chosen from Y and F,
- X₆ is chosen from W and Y,
- X₁₁ is chosen from R and K,
- X₁₂ is chosen from K and Q, and
- X₁₄ is K.

In another embodiment of the invention, the peptide has the sequence DX₃NPX₆LLFLX₁₁X₁₂PX₁₄ (SEQ ID NO: 4) in which:
- X₃ is chosen from Y, F and M,
- X₆ is chosen from W and Y,
- X₁₁ is chosen from R, K and L,
- X₁₂ is chosen from K and Q, and
- X₁₄ is chosen from K and A.

In another embodiment of the invention, the peptide comprises an amino acid sequence, or has an amino acid sequence, selected among those mentioned in table 1 below.

**Table 1**

| Sequence | Reference number |
|---|---|
| VDYNPWLLFLRKPKQ | SEQ ID NO: 6 |
| IDYNPYLLFLKQPKQ | SEQ ID NO: 7 |
| WDMNPWLLFLKLPRQ | SEQ ID NO: 9 |
| LDFNPWLLFLKLPAQ | SEQ ID NO: 8 |
| VDFNPWLLFLKLPAQ | SEQ ID NO: 10 |
| MDFNPWLLFLKVPAQ | SEQ ID NO: 11 |
| VDFNPWLLFLKVPAQ | SEQ ID NO: 12 |
| QDYNPYLLFLKKPKQ | SEQ ID NO: 13 |
| MDFNPYLLFLKKPKQ | SEQ ID NO: 14 |
| YDINPYLLFLKKPQQ | SEQ ID NO: 15 |
| MDFNPYLLFLRKPSQ | SEQ ID NO: 16 |
| MDFNPWMLFLKLPAQ | SEQ ID NO: 17 |
| WDYNPWLLFLRQPQQ | SEQ ID NO: 18 |
| LDFNPYLIFLKMPAQ | SEQ ID NO: 19 |
| LDFNPWFVFLKVPAQ | SEQ ID NO: 20 |
| IDFNPYLLFLKVPAQ | SEQ ID NO: 21 |
| MDYNPWLLFLVQPPQ | SEQ ID NO: 22 |
| IDYNPYLLFLRKPQQ | SEQ ID NO: 23 |
| YDYNPWLLFLRQPTQ | SEQ ID NO: 24 |
| MDFNPWLLFLKLPAQ | SEQ ID NO: 25 |
| VDFNPWLVFLKLPAQ | SEQ ID NO: 26 |
| VDFNPWLLFLKFPAQ | SEQ ID NO: 27 |
| VDFNPYLLFLKLPAQ | SEQ ID NO: 28 |
| LDFNPWLLFLKMPAQ | SEQ ID NO: 29 |
| LDFNPWLLFLKVPAQ | SEQ ID NO: 30 |

"Comprising" means that the above-mentioned sequences may have at least one other amino acid added at the N-terminus, the C-terminus, or both, wherein the resulting peptide retains antiviral activity. For example, at least one linker, for example having two to twenty amino acids, may be added at the N-terminus, the C-terminus, or both. The linker may be added in example for connecting the peptide to another peptide or to a protein, to enhance the stability of the peptide of the invention, to control peptide release, or to optimize its interaction with a target receptor. In another example, one to four additional amino acids may be added at the N-terminus, the C-terminus, or both. Such additional amino acids may be for example "MA" (i.e., methionine-alanine) extension at the N-terminus of the peptide, "GGG" extension at N-terminus, or "GGSK" (SEQ ID NO: 83) extension at C-terminus. Advantageously, these are flexible linkers, which may separate the peptide from other functional sequences, such as tags or fluorophores).

Whatever the embodiment mentioned above, antiviral activity as defined above, i.e. influenza virus polymerase inhibition, is retained.

The peptide of the invention may be synthesized by any method known in the state of the art. It may be a chemical synthesis, such as Solid-Phase Peptide Synthesis (SPPS), such as described by Behrendt et al. ([1]), Liquid-Phase Peptide Synthesis (LPPS), such as described by Sharma et al. ([11]), or a recombinant expression, such as described by Rosano and Ceccarelli ([2]), this list not being exhaustive.

Advantageously, the peptide of the invention may be conjugated to a labeling group or fused to a soluble protein such as green fluorescent protein (GFP), glutathione S-transferase (GST), this list not being exhaustive. The labeling group may be anyone known in the state of the art, for example biotin, fluorophores, such as FAM or FITC, radioisotopes, this list not being exhaustive.

Another object of the invention relates to a nucleic acid encoding a peptide as defined in any one of the preceding claims. According to the invention, nucleic acid refers to DNA or RNA, comprising mRNA (messenger ribonucleic acid). The skilled person can deduce the nucleotide sequence of a peptide thanks to the genetic code, by associating each amino acid with one or more triplets of nucleotides (codons). For illustrative purposes only, "Observed DNA sequence(s)" and "Experimental DNA sequence(s)", i.e. actual sequence of nucleotides that have been detected or measured directly through an experiment or sequencing process or sequence that is derived or expected based on the experimental design or method being used, respectively, are shown in Table 2 below. Furthermore, The skilled person knows that he can adapt these nucleotide sequences according to the organism in which peptide expression is desired, for example by choice of codon. The skilled person knows how to produce the required alternately coded genes for each amino acid toward the codons most commonly found in a host organism, for example as described in Young et al. ([12]).

**Table 2**

| Protein sequence | Observed DNA sequence(s) | Experimental DNA sequence(s) |
|---|---|---|
| VDYNPWLLFLRKPKQ (SEQ ID NO: 6) | No DNA sequences were observed; they were directly synthesised as a peptide for BLI testing. | GTGGATTATAACCCGTGGCTGCTGTTTCTGCGCAAACCGAAACAG (SEQ ID NO: 66) |
| | | GTGGACTACAACCCCTGGCTGCTGTTCCTGAGAAAGCCCAAGCAG (SEQ ID NO: 67) |
| IDYNPYLLFLKQPKQ (SEQ ID NO: 7) | ATCGATTACAACCCGTACCTGCTGTTTCTGAAGTAGCCGAAGCAG (SEQ ID NO: 31) | ATTGATTATAACCCGTATCTGCTGTTTCTGAAACAGCCGAAACAG (SEQ ID NO: 68) |
| | | ATCGACTACAACCCCTACCTGCTGTTCCTGAAGCAGCCCAAGCAG (SEQ ID NO: 69) |
| WDMNPWLLFLKLPRQ (SEQ ID NO: 9) | No DNA sequences were observed; they were directly synthesised as a peptide for BLI testing. | TGGGATATGAACCCGTGGCTGCTGTTTCTGAAACTGCCGCGCCAG (SEQ ID NO: 70) |
| | | TGGGACATGAACCCCTGGCTGCTGTTCCTGAAGCTGCCCAGACAG (SEQ ID NO: 71) |
| LDFNPWLLFLKLPAQ (SEQ ID NO: 8) | TTGGATTTCAATCCGTGGTTGTTGTTCTTGAAATTGCCGGCGCAG (SEQ ID NO: 32) | CTGGATTTTAACCCGTGGCTGCTGTTTCTGAAACTGCCGGCGCAG (SEQ ID NO: 72) |
| | | TTGGATTTCAATCCGTGGTTGTTGTTCTTGAAATTGCCGGCGCAG (SEQ ID NO: 73) |
| VDFNPWLLFLKLPAQ (SEQ ID NO: 10) | GTGGATTTCAATCCGTGGTTGTTGTTCTTGAAATTGCCGGCGCAG (SEQ ID NO:33) | GTGGACTTCAACCCCTGGCTGCTGTTCCTGAAGCTGCCCGCCCAG (SEQ ID NO: 74) |
| | GTCGATTTCAATCCGTGGTTGTTGTTCTTGAAATTGCCGGCGCAG (SEQ ID NO: 34) | |
| MDFNPWLLFLKVPAQ (SEQ ID NO: 11) | ATGGATTTCAATCCGTGGTTGTTGTTCTTGAAAGTGCCGGCGCAG (SEQ ID NO: 35) | ATGGACTTCAACCCCTGGCTGCTGTTCCTGAAGGTGCCCGCCCAG (SEQ ID NO: 75) |
| | ATGGATTTCAATCCGTGGTTGTTGTTCTTGAAAGTCCCGGCGCAG (SEQ ID NO: 36) | |
| VDFNPWLLFLKVPAQ (SEQ ID NO: 12) | GTGGATTTCAATCCGTGGTTGTTGTTCTTGAAAGTCCCGGCGCAG (SEQ ID NO: 37) | GTGGACTTCAACCCCTGGCTGCTGTTCCTGAAGGTGCCCGCCCAG (SEQ ID NO: 76) |
| | GTGGATTTCAATCCGTGGTTGTTGTTCTTGAAAGTGCCGGCGCAG (SEQ ID NO: 38) | |
| QDYNPYLLFLKKPKQ (SEQ ID NO: 13) | TAGGATTACAACCCGTACCTGCTGTTTCTGAAGAAGCCGAAGCAG (SEQ ID NO: 39) | |
| MDFNPYLLFLKKPKQ (SEQ ID NO: 14) | ATGGATTTCAACCCGTACCTGCTGTTTCTGAAGAAGCCGAAGCAG (SEQ ID NO: 40) | |
| YDINPYLLFLKKPQQ (SEQ ID NO: 15) | TACGATATCAACCCGTACCTGCTGTTTCTGAAGAAGCCGTAGCAG (SEQ ID NO: 41) | |
| MDFNPYLLFLRKPSQ (SEQ ID NO: 16) | ATGGATTTCAACCCGTACCTGCTGTTTCTGAGGAAGCCGAGCCAG (SEQ ID NO: 42) | |
| | ATGGATTTCAACCCGTACCTGCTGTTTCTGCGGAAGCCGAGCCAG (SEQ ID NO: 43) | |
| MDFNPWMLFLKLPAQ (SEQ ID NO: 17) | ATGGATTTCAATCCGTGGATGTTGTTCTTGAAATTGCCGGCGCAG (SEQ ID NO: 44) | |
| WDYNPWLLFLRQPQQ (SEQ ID NO: 18) | TGGGATTACAACCCGTGGCTGCTGTTTCTGAGGTAGCCGCAGCAG (SEQ ID NO: 45) | |
| | TGGGATTACAACCCGTGGCTGCTGTTTCTGAGGCAGCCGTAGCAG (SEQ ID NO: 46) | |
| LDFNPYLIFLKMPAQ (SEQ ID NO: 19) | TTGGATTTCAATCCGTACTTGATCTTCTTGAAAATGCCGGCGCAG (SEQ ID NO: 47) | |
| LDFNPWFVFLKVPAQ (SEQ ID NO: 20) | TTGGATTTCAATCCGTGGTTCGTCTTCTTGAAAGTCCCGGCGCAG (SEQ ID NO: 48) | |
| | TTGGATTTCAATCCGTGGTTCGTGTTCTTGAAAGTGCCGGCGCAG (SEQ ID NO: 49) | |
| | TTGGATTTCAATCCGTGGTTCGTCTTCTTGAAAGTGCCGGCGCAG (SEQ ID NO: 50) | |
| | TTGGATTTCAATCCGTGGTTCGTGTTCTTGAAAGTCCCGGCGCAG (SEQ ID NO: 51) | |
| IDFNPYLLFLKVPAQ (SEQ ID NO: 21) | ATCGATTTCAATCCGTACTTGTTGTTCTTGAAAGTCCCGGCGCAG (SEQ ID NO: 52) | |
| MDYNPWLLFLVQPPQ (SEQ ID NO: 22) | ATGGATTACAACCCGTGGCTGCTGTTTCTGGTGTAGCCGCCCCAG (SEQ ID NO: 53) | |
| IDYNPYLLFLRKPQQ (SEQ ID NO: 23) | ATCGATTACAACCCGTACCTGCTGTTTCTGAGGAAGCCGTAGCAG (SEQ ID NO: 54) | |
| | ATCGATTACAACCCGTACCTGCTGTTTCTGCGCAAGCCGTAGCAG (SEQ ID NO: 55) | |
| YDYNPWLLFLRQPTQ (SEQ ID NO: 24) | TACGATTACAACCCGTGGCTGCTGTTTCTGCGCTAGCCGACCCAG (SEQ ID NO: 56) | |
| MDFNPWLLFLKLPAQ (SEQ ID NO: 25) | ATGGATTTCAATCCGTGGTTGTTGTTCTTGAAATTGCCGGCGCAG (SEQ ID NO: 57) | |
| VDFNPWLVFLKLPAQ (SEQ ID NO: 26) | GTGGATTTCAATCCGTGGTTGGTGTTCTTGAAATTGCCGGCGCAG (SEQ ID NO: 58) | |
| | GTGGATTTCAATCCGTGGTTGGTCTTCTTGAAATTGCCGGCGCAG (SEQ ID NO: 59) | |
| VDFNPWLLFLKFPAQ (SEQ ID NO: 27) | GTGGATTTCAATCCGTGGTTGTTGTTCTTGAAATTCCCGGCGCAG (SEQ ID NO: 60) | |
| VDFNPYLLFLKLPAQ (SEQ ID NO: 28) | GTGGATTTCAATCCGTACTTGTTGTTCTTGAAATTGCCGGCGCAG (SEQ ID NO: 61) | |
| LDFNPWLLFLKMPAQ (SEQ ID NO: 29) | TTGGATTTCAATCCGTGGTTGTTGTTCTTGAAAATGCCGGCGCAG (SEQ ID NO: 62) | |
| LDFNPWLLFLKVPAQ (SEQ ID NO: 30) | TTGGATTTCAATCCGTGGTTGTTGTTCTTGAAAGTGCCGGCGCAG (SEQ ID NO: 63) | |
| | TTGGATTTCAATCCGTGGTTGTTGTTCTTGAAAGTCCCGGCGCAG (SEQ ID NO: 64) | |
| | TTGGATTTCAATCCGTGGTTGTTGTTCTTGAAAGTCCCGGCACAG (SEQ ID NO: 65) | |

Another object of the invention relates to an expression vector into which the above-mentioned nucleic acid may be inserted. Suitable expression vectors are known by the skilled person, and it may be for example selected among pET, such as pET-28a, pET-21a for His-tagged proteins, pGEX, pcDNA, pCMV or pYES, this list not being limitative. The expression vector may comprise typical elements for expression of the peptide, depending on the system of expression. It may have for example elements comprising a promoter, such as T7 or CMV, a ribosome-binding site, cloning sequences, terminator sequences, selection marker, such as ampicillin or kanamycin, an origin of replication, a protein fusion tag, such as GFP tag or His tag and/or a signal sequence, this list not being limitative.

Another object of the invention relates to a cell transfected or modified genetically with an expression vector as defined above. Suitable cells for expressing the peptide are known by the skilled person, and it may be for example selected among such as BHK-21, E. coli, Pichia pastoris, Sf9, Sf21, HEK293, HeLa, COS, NIH-3T3, or CHO. Suitable cells for genetic modification are also known by the skilled person, and may be for example previously cited cells, or non-human animal cells, preferably non-human embryonic cells or non-human germinal stem cells (human embryonic stem cells are excluded), such as poultry, for example chicken, turkey, duck, geese, ostrich, horse, pig, cattle, for example dairy cattle of beef cattle, including cows, bulls and steers, sheep, for example lamb or mutton, goat or rabbit. Advantageously, genetic modification involves a stable and heritable modification of the genome, to create organisms with specific characteristics, especially flu resistance. Techniques allowing obtention of such genetically modified cells are described in the state of the art, for example by Intarapat and Stern ([13]) or Bahrami et al. ([14]).

Another object of the invention relates to a vector for delivery of a nucleic acid as defined above. The vector for delivery may be any vector known in the state of the art. It may be for example a Lipid Nanoparticle (LNP) or a Tat cell-penetrating peptide, preferably LNP. Preferably, the vector for delivery is LNP, delivering mRNA as defined above. Indeed, the inventors have demonstrated that the mRNA-LNP preparations, in which the mRNA encoding the peptides is delivered into the cells, inhibit influenza A virus replication in infection assays 1000 times more than a negative control and 100 times more than the wild-type (WT) peptide.

Another object of the invention relates to a medicament, also referred to as a "pharmaceutical composition", comprising a peptide as defined above, or a nucleic acid as defined above, or a vector as defined above.

Another object of the invention relates to a peptide as defined above, or a nucleic acid as defined above, or a vector as defined above, for use as a medicament.

The medicament or pharmaceutical composition may comprise as sole active ingredient the peptide as defined above, or the nucleic acid as defined above, or the vector as defined above. Alternatively, it may comprise at least one other active ingredient. In any case, it may contain at least one pharmaceutically acceptable diluent and/or excipient. As used herein, the term "pharmaceutically acceptable" refers to physiologically tolerable, for either human or veterinary application. Similarly, "medicament" or "pharmaceutical composition" include formulations for human and veterinary use, such as for poultry, horse, pig, cattle, sheep, goat or rabbit or other animals susceptible to influenza. If present, the at least one additional active ingredient may be any therapeutic ingredient that is known to the person skilled in the art for treating or preventing the pathology of interest. Nonlimiting examples include antiviral agents such as oseltamivir, zanamivir, baloxavir marboxil, or peramivir; antibiotics such as azithromycin or amoxicillin-clavulanate; or immunomodulatory agents such as corticosteroids.

It can be administered before, during or after the peptide of the invention. When two or more active ingredients are to be administered, they can be administered in the same pharmaceutical composition or in separate pharmaceutical compositions. When administered in separate pharmaceutical compositions, they can be administered simultaneously or one can be administered first. The amount of time between administration of the different compounds can vary and can be determined by one of ordinary skill in the art.

For example, suitable formulations and effective dose of the active principle(s) may be derived from the formulation of known antiviral molecules, such as oseltamivir, zanamivir, baloxavir marboxil, or peramivir.

Another object of the invention relates to the peptide as defined above, or the nucleic acid as defined above, or the vector as defined above, for use in the treatment of influenza. The terms "treatment" and "treating" refers to any effect, e.g., lessening, reducing, modulating, ameliorating, reversing, or eliminating the condition, that may result in the improvement of the condition, disease, disorder, and the like, or ameliorating a symptom thereof. Those in need of treatment include those already with the condition or symptoms, as well as those having the conditions but not the symptoms yet. Alternatively, healthy individuals can be treated in a preventative manner, using the medicament in a prophylactic manner.

Another object of the invention is a method for preparing a peptide as defined above, comprising the chemical synthesis of the peptide as described above or the recombinant expression of the peptide as described above.

This invention is further illustrated by the following examples with regard to the annexed drawings that should not be construed as limiting.

### Brief description of the figures

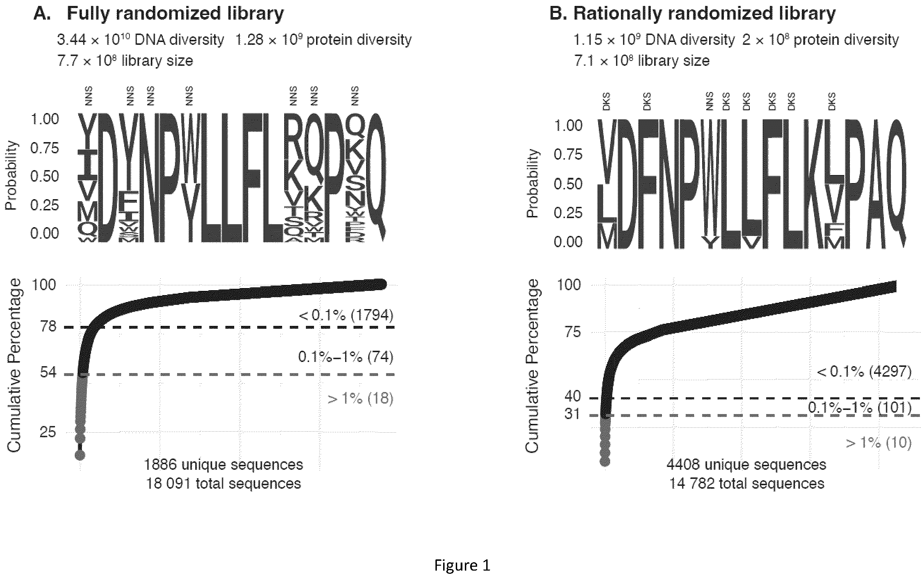

- Figure 1: represents the comparison of library design strategies using phage display sequencing data. Amino acid distributions are shown as sequence logos (top), with "NNS" or "DKS" above indicating the positions where the degenerate codon randomization strategies were applied. Cumulative percentage plots (bottom) display the frequency of unique sequences in each library following multiple rounds of affinity selection according to a phage display protocol. Key frequency thresholds (>1%, 0.1-1%, <0.1%) are highlighted. (A) FR library: Theoretical diversity of 3.44 × 10¹⁰ DNA and 1.28 × 10⁹ protein sequences; library size of 7.7 × 10⁸. Out of 18,091 sequences, 1886 were unique, with 18 >1%, 74 between 0.1-1%, and 1794 <0.1%. (B) RR library: Theoretical diversity of 1.15 × 10⁹ DNA and 2 × 10⁸ protein sequences; library size of 7.1 × 10⁸. Out of 14,782 sequences, 4408 were unique, with 10 >1%, 101 between 0.1-1%, and 4297 <0.1%.

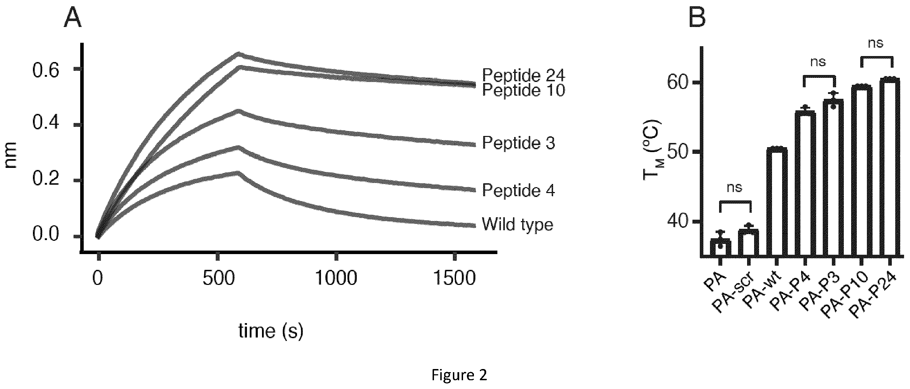

- Figure 2: represents the binding affinity and stability of selected peptides. (A) Bio-Layer Interferometry (BLI) sensorgrams showing the binding kinetics of selected peptides (Peptides 3, 4, 10, 24) and wild type to the PA subunit, measured as a change in nm over time. (B) Thermal shift assay (Tₘ) showing the melting temperatures of peptide-PA complexes. Unless marked as not significant (ns), differences between peptides are statistically significant.

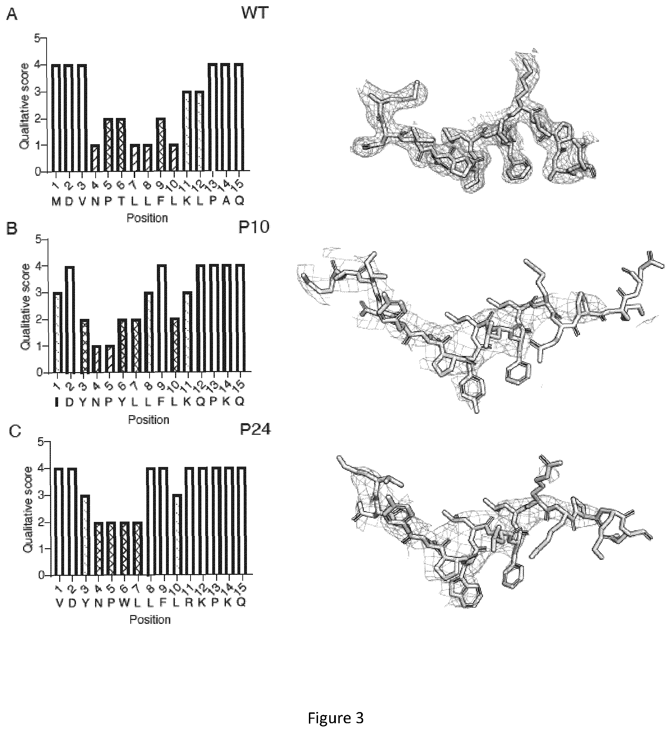

- Figure 3: represents the structural analysis of peptide-PA interactions. Qualitative binding scores for single alanine substitutions at each position of each peptide revealing hot spots of interaction (left) and corresponding crystal structures (right) are shown for the interactions of the PA subunit with (A) wild-type peptide, (B) peptide 10, and (C) peptide 24. The y axis of the bar plot represents the qualitative binding scores at each residue position of the peptides. Level 4 for a similar binding as the control, 3 for mild impairment, 2 for a moderate, and 1 for severe impairment or complete abrogation of the interaction. To the right, the crystal structures depict the peptide and their corresponding 1 sigma electron density is depicted.

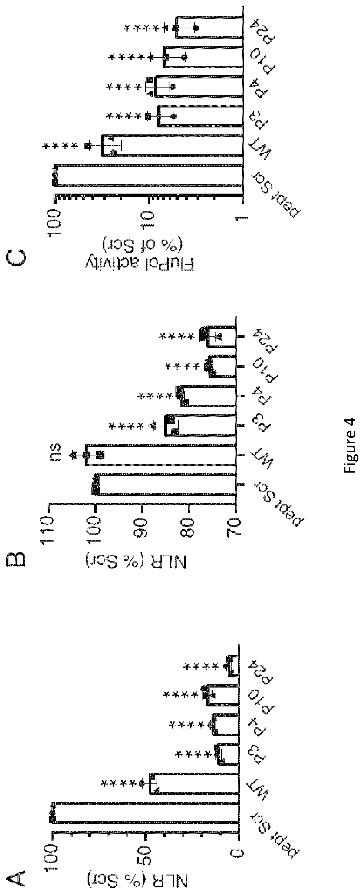

- Figure 4: represents the functional inhibition assays. (A, B) Split-luciferase assay showing the disruption of PA-PB1 interaction in influenza A (A) and influenza B (B) by wild-type and selected peptides (P3, P4, P10, P24). The normalized luciferase ratio (NLR) is expressed as a percentage of the scrambled peptide control (pept Scr) that comprises a sequencescrambled version of the wild-type peptide with similar molecular weight but different primary sequence. (C) Minireplicon assay showing reduced polymerase activity in the presence of wild-type and selected peptides. Statistical significance is indicated: p < 0.05 (*), p < 0.01 (**), p < 0.001 (***), and p < 0.0001 (****); ns, not significant.

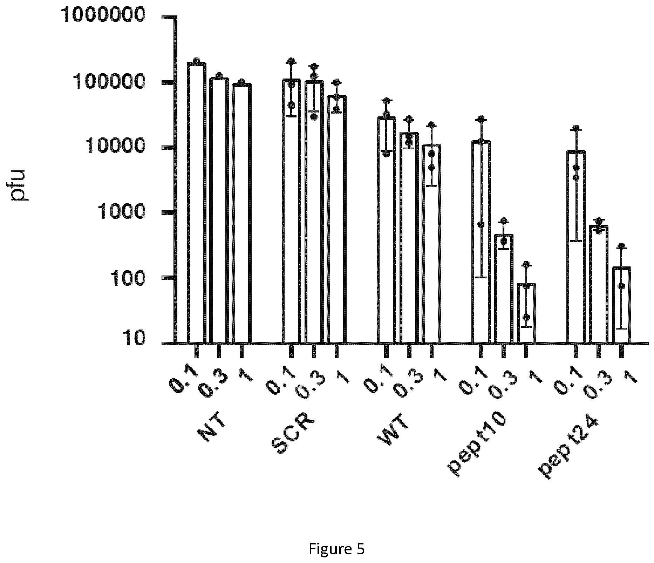

- Figure 5: represents the antiviral activity of peptide-encoding mRNA-LNPs. Dose-dependent reduction in viral titers (measured as plaque-forming units, pfu) for H1N1 influenza following treatment with mRNA-LNPs encoding wild-type peptide (WT), peptide 10, or peptide 24. NT represents the no-treatment control, and SCR represents the scrambled peptide control. Peptides were tested at concentrations of 0.1, 0.3, and 1 dose-units.

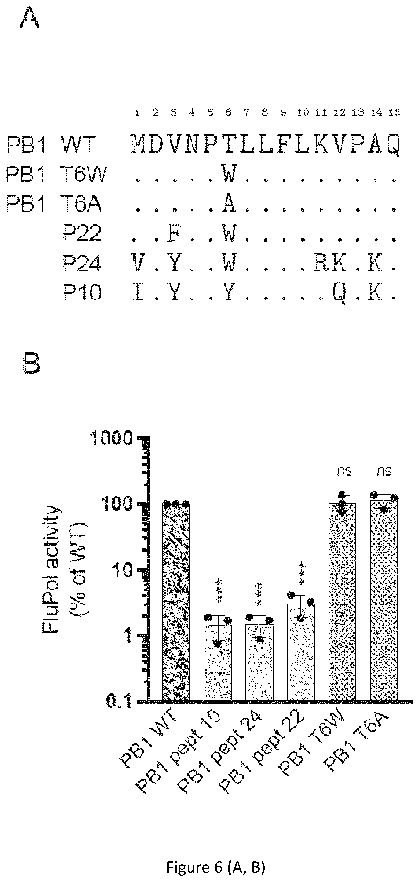

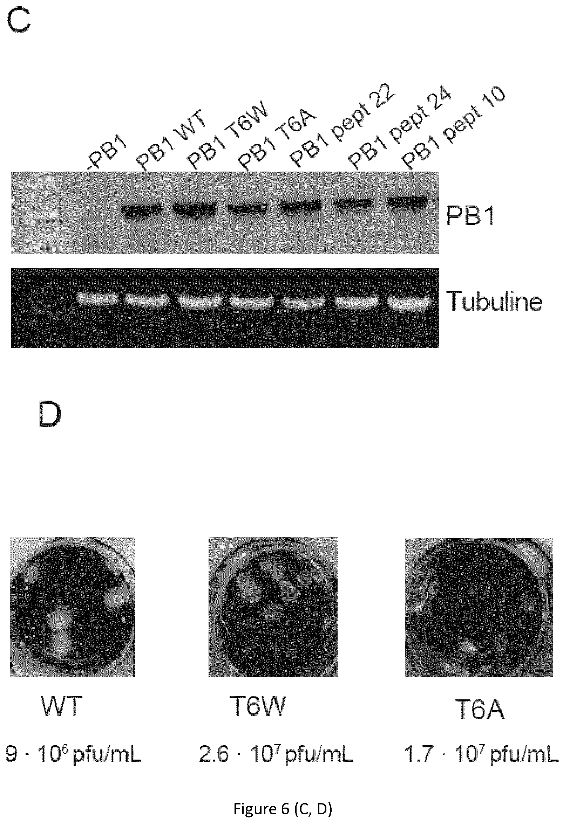

- Figure 6: represents mutational impact on polymerase function. (A) Sequence alignment of the PB1 N-terminal region showing wild-type (WT) PB1 and specific mutations (T6W, T6A, P22, P24, P10). (B) Polymerase activity assay of reconstituted FluPol showing the effect of mutations and peptide insertions on polymerase function, expressed as a percentage of WT activity. Peptide insertions P10, P24, and P22 significantly reduce activity, while T6W and T6A mutations have no significant effect (ns). **p < 0.001. (C) Western blot analysis confirming PB1 expression levels in cells transfected with WT PB1, mutants (T6W, T6A), or peptide insertions (P22, P24, P10). Tubulin is shown as a loading control. (D) Viral recovery assay using reverse genetics, showing plaque formation and viral titers (pfu/mL) for WT, T6W, and T6A PB1 variants.

### Examples

### Example 1: Identification of affinity-enhanced peptides capable of disrupting the interaction between the influenza virus polymerase subunits PA and PB1

Phage display directed evolution was employed to identify affinity-enhanced peptides capable of disrupting the interaction between the influenza virus polymerase subunits PA and PB1-a critical association for viral replication. Utilizing mRNA lipid nanoparticle technology for intracellular delivery, we demonstrate effective inhibition of viral replication in vitro without observable toxicity.

### Screening of peptides disrupting the PA-PB1 interaction through optimized phage display and next-generation sequencing

Two phage display attempts were conducted to identify peptides disrupting the PA-PB1 interaction. The first attempt, using a classical protocol with ELISA and Sanger sequencing to characterize selected clones, yielded a library size of 2 × 10⁵ colonies, from which four potential peptide candidates were identified. A second optimized attempt, which incorporated improved transformation techniques and Next Generation Sequencing (NGS), resulted in significantly larger libraries, with a size of 7.7 × 10⁸ for the fully randomized (FR) library comprising NNS codons and 7.1 × 10⁸ for the rationally randomized (RR) library comprising more limited degenerate codons such as DKS (Figure 1A, B). Approximately 200,000 NGS sequence reads were obtained from each library, with 14,000-18,000 high-quality reads post processing.

Abundance calculations after three rounds of affinity selection on immobilized PA C-ter domain revealed that the RR library exhibited greater diversity, containing 4,408 unique sequences compared to 1,886 unique sequences in the FR library. The FR library displayed a higher concentration of dominant sequences, with 18 sequences comprising 54% of the total reads, whereas the RR library had only 10 sequences accounting for 31% of the total reads.

Sequence alignment of the sequences more abundant than 1% showed distinct patterns: the FR library favoured tyrosine or isoleucine/valine at the N-terminus and charged residues at the C-terminus, whereas the RR library displayed less variability in amino acid distribution (Fig. 1A, B), with the hydrophobic amino acids valine, leucine or methionine preferred on the N-terminus. Based on frequency and clustering analysis, 19 peptide sequences were selected as potential hits, with one additional peptide (peptide 24) rationally designed by identifying common residues across different phage display selected sequences.

### Biophysical characterization of high-affinity peptides reveals enhanced binding and stability

All peptide candidates were evaluated for binding affinity using Bio-Layer Interferometry (BLI), with the two top peptides from each phage display assessed side-by-side.

Experimental results of peptide sequences are shown in Table 2. All experimental sequences, plus all sequences having more than 1% representation from the total sequences recovered from NGS. The wild-type peptide had a K_{D} between 20-30 nM (as a reference).

**Table 2**

| Sequence | Reference number | Affinity (K_{D} in nM) by BLI | PCA |
|---|---|---|---|
| VDYNPWLLFLRKPKQ | SEQ ID NO: 6 | 0.16 | Yes |
| IDYNPYLLFLKQPKQ | SEQ ID NO: 7 | 0.36 | Yes |
| WDMNPWLLFLKLPRQ | SEQ ID NO: 9 | 3.9 | Yes |
| LDFNPWLLFLKLPAQ | SEQ ID NO: 8 | 2.7 | Yes |
| VDFNPWLLFLKLPAQ | SEQ ID NO: 10 | 0.32 | Yes |
| MDFNPWLLFLKVPAQ | SEQ ID NO: 11 | 0.28 | Yes |
| VDFNPWLLFLKVPAQ | SEQ ID NO: 12 | 0.24 | Yes |
| QDYNPYLLFLKKPKQ | SEQ ID NO: 13 | 1.12 | |
| MDFNPYLLFLKKPKQ | SEQ ID NO: 14 | 0.64 | |
| YDINPYLLFLKKPQQ | SEQ ID NO: 15 | 1.25 | |
| MDFNPYLLFLRKPSQ | SEQ ID NO: 16 | 0.93 | |
| MDFNPWMLFLKLPAQ | SEQ ID NO: 17 | 0.83 | |
| WDYNPWLLFLRQPQQ | SEQ ID NO: 18 | 1.00 | |
| LDFNPYLIFLKMPAQ | SEQ ID NO: 19 | 0.84 | |
| LDFNPWFVFLKVPAQ | SEQ ID NO: 20 | 1.59 | |
| IDFNPYLLFLKVPAQ | SEQ ID NO: 21 | 0.27 | |
| MDYNPWLLFLVQPPQ | SEQ ID NO: 22 | Better than WT | |
| IDYNPYLLFLRKPQQ | SEQ ID NO: 23 | Better than WT | |
| YDYNPWLLFLRQPTQ | SEQ ID NO: 24 | Better than WT | |
| MDFNPWLLFLKLPAQ | SEQ ID NO: 25 | Better than WT | |
| VDFNPWLVFLKLPAQ | SEQ ID NO: 26 | Better than WT | |
| VDFNPWLLFLKFPAQ | SEQ ID NO: 27 | Better than WT | |
| VDFNPYLLFLKLPAQ | SEQ ID NO: 28 | Better than WT | |
| LDFNPWLLFLKMPAQ | SEQ ID NO: 29 | Better than WT | |
| LDFNPWLLFLKVPAQ | SEQ ID NO: 30 | Better than WT | |

From the first phage display, peptide 3 exhibited an 8.7-fold increase in affinity compared to the wild-type peptide, with a dissociation constant (K_{d}) of 3.9 nM versus 33.8 nM for the wild type. Peptide 4 showed a 3.7-fold improvement, with a K_{d} of 9.1 nM. In the second phage display, peptides 10 and 24 displayed the highest affinities, with K_{d} values of 3.4 nM and 3.8 nM, representing 9.9-fold and 8.9-fold improvements over the wild type, respectively. All four peptides demonstrated slower dissociation rates (k_{off}) compared to the wild type, explaining the basis of the higher affinity values, as reflected in the flatter dissociation curves (Figure 2 A).

Association rate constants (kₒₙ) for peptides 3 and 4 were 56,690 M⁻¹ s⁻¹ and 49,260 M⁻¹ s⁻¹, respectively, compared to 62,400 M⁻¹ s⁻¹ for the wild type (Table 1). Peptides 10 and 24 had kₒₙ values of 28,840 M⁻¹ s⁻¹ and 44,740 M⁻¹ s⁻¹, respectively. Half-lives (T_{1/2}) of dissociation of the complexes were longest for peptides 10 and 24, at 6,931 s and 3,465 s, respectively, compared to 577 s for the wild type. Peptides 3 and 4 exhibited half-lives of 3,465 s (Table 3).

Thermal Shift Assay (TSA) analysis revealed an increase in melting temperature (Tₘ) for all selected peptides relative to controls (Figure 2 B). The wild-type peptide showed a 10°C increase in Tₘ relative to the negative controls, while peptides 3, 4, 10, and 24 exhibited an increase of approximately 20°C. No significant differences were observed between peptides 3 and 4, or between peptides 10 and 24; however, a significant difference between these two groups placed peptides 10 and 24 as having the highest Tₘ values.

**Table 3**

| Sequence | Name | Kd (nM) | kₒₙ (M⁻¹ s^{- 1}) | k_{off} (s⁻¹) | T_{1/2} (s) | Tₘ (°C) |
|---|---|---|---|---|---|---|
| MDVNPTLLFLKVP AQ (SEQ ID NO: 5) | WT | 17.6 | 62 400 | 0.0012 | 577 | 37.5 |
| LDFNPWLLFLKLP AQ (SEQ ID NO: 8) | P3 | 2.7 | 56 690 | 0.0002 | 3 465 | 57.5 |
| WDMNPWLLFLKL PRQ (SEQ ID NO: 9) | P4 | 3.9 | 49 260 | 0.0002 | 3 465 | 55.8 |
| IDYNPYLLFLKQP KQ (SEQ ID NO: 7) | P10 | 0.36 | 28 840 | 0.0001 | 6 931 | 59.5 |
| VDYNPWLLFLRK PKQ (SEQ ID NO : 6) | P24 | 0.16 | 44 740 | 0.0002 | 3 465 | 60.5 |

### Conserved binding and reduced residue sensitivity is revealed by structural and functional assays

The crystal structures of three out of the four selected peptides were resolved at resolutions between 2.95 Å and 3.56 Å, showing that binding within the alpha-helix pocket of PA was similar to the wild-type structure (PDB 2ZNL (Obayashi et al. ([3])). In the lower-resolution structures, electron densities for the final 1-2 residues were not observed, suggesting some flexibility in these regions, while the residues within the binding pocket were well-defined. To assess the contribution of individual amino acids to peptide function, we performed alanine scanning mutagenesis. The wild-type peptide showed four positions that were sensitive to alanine substitution (Figure 3 A). In contrast, peptide 10 showed sensitivity at only two positions (Figure 3 B), while peptide 24 did not display any critical positions (Figure 3 C). Notably, nine out of the fifteen residues in peptide 24 were unaffected by alanine substitution, compared to six unaffected residues in both the wild-type peptide and peptide 10. Specifically, positions 8 to 10 appeared less affected in peptides 10 and 24 compared to the wild type, despite all having the same amino acid sequence (L-F-L) at these positions. The reasoning for this observation is that the weaker binding wild-type peptide is more sensitive to Ala substitutions, whereas the tighter binding variants are able to absorb Ala substitutions without strongly damaging the interaction.

### Effective in-vitro disruption of PA-PB1 interaction and polymerase activity

Plasmid-based assays demonstrated disruption of the PA-PB1 interaction of both Influenza A and Influenza B polymerases, as assessed by the split-luciferase assay (Figure 4 A, B). In cells expressing the wild-type plasmid, a 50% reduction of the PA-PB1 interface was observed for Influenza A polymerase, while no significant reduction was detected for Influenza B polymerase. All four selected peptides resulted in a greater disruption of the interface, showing approximately 90% reduction for Influenza A. For Influenza B, peptides 3 and 4 caused a lower disruption, while peptides 10 and 24 exhibited similar levels of disruption to those observed for Influenza A. In the minireplicon assay, a decrease in influenza A FluPol activity was observed (Figure 4 C). The wild-type plasmid led to a 30% reduction in FluPol activity, while all four peptides showed a consistent reduction of approximately 90%. No statistically significant differences were observed in the disruption of the PA-PB1 interaction or polymerase activity across the four selected peptides.

No toxicity was observed for any of the peptides. Additionally, there was no reduction in the interaction of unrelated proteins Jun-Fos and Gaussia FL with any of the peptides, confirming the specificity of the peptide mechanism.

### Innovative mRNA-Lipid Nanoparticle delivery achieves potent antiviral activity

Successful intracellular delivery and significant antiviral activity using mRNA lipid nanoparticles (mRNA-LNPs) encoding the selected peptides was observed. A dose-dependent logarithmic decrease in plaque-forming units (pfu) was observed 24 hours post-infection with influenza A H1N1 and H3N2 strains following the application of peptide-encoding mRNA-LNPs. The mRNA-LNPs encoding the wild-type peptide exhibited a 2- to 10-fold reduction in viral activity compared to both untreated cells and the negative control. Notably, peptides 10 and 24 demonstrated a stronger antiviral response, achieving the same reduction in pfu as the wild-type peptide but at doses ten times lower. Specifically, for the wild type, one dose unit resulted in 10⁵ pfu, whereas peptides 10 and 24 produced the same pfu at 0.1 dose units-a tenfold lower dose. At one dose unit, peptides 10 and 24 resulted in only 100 pfu, representing a 1,000-fold reduction compared to controls and a 100-fold reduction compared to the wild type at the same dose (Figure 5).

### High-affinity PB1_{Nter} peptides impair polymerase function when incorporated into full-length PB1

The impact of introducing mutations corresponding to peptides 10 and 24 into the PB1_{Nter} region in the context of the full heterotrimeric FluPol on polymerase activity and viral recovery was evaluated. Peptide 10 contains five mutations, while peptide 24 has six mutations. An additional peptide was included, a double mutant called peptide 22 which replaced threonine at position 6 with tryptophan and valine at position 3 with phenylalanine (Figure 6A). While these mutations resulted in significantly higher binding affinities to the PA subunit, the corresponding PB1 mutants when introduced into full-length PB1 exhibited a drastic reduction in polymerase activity in a minireplicon assay -to around 1% of the wild-type level-and prevented viral recovery (Figure 6 B, D). In contrast, single-point mutations at position 6 (T6W and T6A, viral ribonucleoproteins generated with the PB1 peptide in which position 6 was mutated to Trp or Ala respectively) did not affect polymerase activity or viral recovery, displaying plaque morphologies like those of the wild-type virus (Figure 6 D). Western blot analysis confirmed that all PB1 variants were expressed at similar levels (Fig. 6 C).

### Discussion

In this study, we identified high-affinity peptides that disrupt the critical interaction between influenza virus polymerase subunits PA and PB1, thereby inhibiting viral replication. The use of a smaller rationally designed phage libraries based on structural information was useful even when traditional methods such as ELISA were employed to characterize hits, as it led to the discovery of peptides 3 and 4. However, utilizing larger and more diverse phage display libraries proved instrumental in discovering peptides with up to a tenfold increase in binding affinity compared to the wild type. There is an advantage in using extensive library sizes for identifying superior binders, fact that has also been proved mathematically. The integration of next generation sequencing (NGS) into our screening workflow provided a comprehensive view of peptide diversity, correlating with classical methods but offering deeper insights. NGS allowed us to identify promising candidates by analyzing sequence abundance and clustering, emphasizing its value in refining peptide selection beyond what traditional ELISA-based approaches offer.

Structural analyses confirmed that the selected peptides bind within the same alpha-helix pocket on the PA subunit as the wild-type peptide (Obayashi *et al.* ([3]); He *et al.* ([4])). However, the unresolved terminal amino acids suggest some flexibility. This indicates that while the core binding mode is conserved, peripheral residues may influence binding dynamics. Alanine scanning mutagenesis revealed that the new peptides showed less dependence on critical positions compared to the wild-type. This underscores the importance of the overall peptide context and interresidue interactions, suggesting that enhancements in one region can mitigate sensitivities in another and that the overall interaction results from multiple cooperative interactions between amino acids along the peptide length with the target PA domain.

Thorough toxicity assessments ensured that the antiviral effects were not confounded by cytotoxicity. Therefore, our functional assays demonstrated that the selected peptides effectively and specifically disrupt the PA-PB1 interaction and significantly reduce polymerase activity without inducing cytotoxicity. The use of mRNA lipid nanoparticles (mRNA-LNPs) for intracellular delivery marks a significant advancement in the field. This method circumvents the challenges of direct peptide administration, such as poor membrane permeability and rapid degradation. By encoding peptides within mRNA-LNPs, we achieved efficient intracellular expression and potent antiviral activity. To our knowledge, this is the first application of mRNA-LNPs for delivering intracellular antiviral peptides, opening new therapeutic avenues previously overlooked in peptide research.

Our viral reconstitution assays provided deeper insights into the relationship between peptide affinity and functional activity. Interestingly, although T6W was reported to decrease polymerase activity by approximately 40% in another study, we did not observe any differences in polymerase activity for this mutant. The complete recovery of the mutant T6A, which had previously shown in our alanine scanning to abrogate the binding to PA, contradicts the idea that there is an optimal range of binding affinity, that when crossed limits the viral growth. Our findings suggest that factors beyond binding affinity-such as structural compatibility or functional relationships between viral and host components-are crucial for maintaining viral replication functionality.

In conclusion, our approach combines phage display, NGS, and advanced delivery systems to develop potent peptide inhibitors against influenza virus. This methodology not only enhances the discovery of high-affinity binders but also addresses historical barriers in peptide therapeutics, such as delivery and stability. By unlocking a new class of antiviral agents, we offer a novel framework for targeting critical protein-protein interactions in various diseases.

### Methods

### PA_{Cter} purification

PA_{Cter} (residues 254-716) from the 1933 Wilson-Smith Influenza A virus H1N1 (A/WS/1933/H1N1, UniProt ID P15659) was expressed as a His-Smt fusion protein. The corresponding DNA was provided in a vector by the Seattle Structural Genomics Centre for Infectious Disease (SSGCID) (Moen et al. ([5])). The DNA encoding this PA_{Cter} fragment was cloned into the pET9a-derived plasmid pESPRIT002 between the Aatll and Nsil restriction sites, resulting in the expression construct: 6His-PACter-BAP (biotin acceptor protein). DNA purification was performed using Macherey-Nagel NucleoSpin DNA kits, and minipreps were carried out with QIAprep Spin Miniprep Kits, following the manufacturers' protocols. E. coli BLI21 Al RIL cells transformed with the His-Smt fusion plasmid were grown in LB medium supplemented with kanamycin (50 mg/mL) at 37°C with shaking until an OD600 of 0.6-0.8 was reached. Protein expression was induced by adding IPTG to a final concentration of 1 mM and arabinose to 0.2% (w/v), followed by incubation at 18°C with shaking overnight. Cells were harvested by centrifugation. Proteins were purified using nickel affinity chromatography, followed by cleavage of the fusion tag with ULP-1 protease. The cleaved protein was subjected to a second round of nickel affinity chromatography to remove the fusion tag, as described previously (Moen et al. ([5])). Final purification was achieved by size-exclusion chromatography using a Superdex 200 Increase 10/300 GL column in a buffer containing 200 mM NaCl, 25 mM Tris pH 8.0, 1% (v/v) glycerol, 10 mM β-mercaptoethanol, and 50 mM RE pH 8.

### Phage Display Library Construction and Screening for PA-PB1 Interaction Peptides

Two phage display libraries were designed based on the structure of the wild-type PA-PB1 interaction (PDB: 2ZNL). The first library, Fully Randomized (FR), utilized NNS degenerate oligonucleotides at seven positions (1, 3, 4, 6, 11, 12, 14), yielding an estimated DNA diversity of 3.44 × 10¹⁰ and protein diversity of 1.28 × 10⁹. The second library, Rationally Randomized (RR), employed DKS degenerate oligonucleotides at positions 1, 3, 7, 8, 9, 10 and NNS at position 6, achieving a DNA diversity of 1.15 × 10⁹ and a protein diversity of 2 × 10⁸. Primers were cloned into the pADL-100 vector between the pelB leader sequence and the plll gene using the Sfil restriction site. A GSG peptide linker was added at the N-terminus. Whole-plasmid PCR cloning techniques were used³⁵ (Kong et al. ([6]), and DNA was purified using Macherey-Nagel NucleoSpin DNA kits, following the manufacturer's instructions. For the library cloning, E. coli Lucigen TG1 cells were electroporated at 4°C using at first 50 ng of plasmid and in the second iteration 1 µg of plasmid per 25 µL of cells. After electroporation, cells were resuspended in 1 mL of pre-chilled recovery medium (37°C) and plated onto Q-tray plates containing 2XYT agar with 100 µg/mL ampicillin and left overnight. Then the library would be scraped, and flash frozen for conservation. Five hundred mL of the resuspended library was grown in liquid LB medium until an OD₆₀₀ of 0.4. Phage production was induced by adding M13KO7d3 helper phage for 1 hour at 37°C, followed by induction with 1 mM IPTG at 30°C overnight with shaking. The phage particles were purified using PEG precipitation (Lee et al.([7]).

For library screening, biotinylated PA was immobilized onto 1 mg of Dynabeads^{™} MyOne^{™} Streptavidin T1 magnetic beads for 4 hours under saturating conditions, with excess PA removed by washing. The phage libraries were incubated with the PA-coated beads overnight to allow binding. Washing was performed with six rounds of PBS containing 0.1% Tween 20, followed by two rounds with PBS. Bound phages were eluted with 1 mL of 1 M glycine-HCl (pH 2.0) for 10 minutes, and the eluate was neutralized with 150 µL of 1 M Tris-HCl (pH 9.0). Eluted phages were then infected into TG1 cells (OD600 = 0.4) for 1 hour at 37°C, and the library size was determined by plating 10-fold dilutions on petri dishes. Two distinct selection strategies were applied: a classical panning procedure and a modified approach incorporating off-rate selection to enhance the isolation of high-affinity binders. The classical selection repeated the process of the first round three times. In the improved approach, for the second round of panning, a 10-fold lower amount of Dynabeads (0.1 mg) was used, and off-rate selection was applied by adding 1 mg of non-biotinylated PA to the incubation to compete off weaker binders for two hours (Zahnd et al. ([8])). After incubation, phages bound to the remaining beads were washed and eluted as before. For the third round, only 0.01 mg of Dynabeads were used, and the rest of the procedure followed the same off-rate selection protocol as in the second round.

### NGS and sequence analysis

A 500 mL volume of scrapped colonies from round 3 from the phage display were mini prepped using QIAprep Spin Miniprep Kits and then performed 25 PCR cycles using Phusion High-Fidelity DNA Polymerase with the following partial Illumina adapter sequences: forward 5'-ACACTCTTTCCCTACACGACGCTCTTCCGATCT-3' (SEQ ID No: 77) and reverse: 5'-GACTGGAGTTCAGACGTGTGCTCTTCCGATCT-3' (SEQ ID No: 78). Those primers surround a 500 bp region of interest from the phagemid. They were cleaned up using Macherey-Nagel NucleoSpin DNA kits and gathered 500 ng of material, normalised to 20 ng/mL. They were sent for NGS sequencing using the amplicon EZ service by Genewiz. The returned csv files were analysed using custom python scripts; briefly, the sequences were trimmed, keeping only a 15 - 110 base pair region with Phred scores of >64, keeping 10-25% of the original sequences. Next, the sequences were matched to the forward adaptor confirming identity and correct sequence register. Finally, the sequences were translated. A frequence analysis was performed by aggregating all the sequences with the same phenotype. Only the sequences repeated 5 or more times were considered in the analysis. Finally, pairwise Hamming distances were calculated using the R package "stringdist", and the sequences were split into 20 clusters.

### BLI

Peptides were synthesised using Solid-Phase Peptide Synthesis (SPPS) (Behrendt et al. ([1])) and delivered either as crude products diluted in 100% DMSO or purified with HPLC and delivered as a dry powder. Quality control was performed by mass spectrometry. BLI experiments were conducted using an OctetRED96e (PALL/Forte Bio) instrument with streptavidin-coated capillary biosensors. Biotinylated peptides were immobilized onto the sensors at varying concentrations to achieve a signal of 1 nm, followed by titration with increasing concentrations of the PA_{Cter} domain to establish the optimal working concentration range. The binding buffer consisted of 200 mM NaCl, 25 mM Tris pH 8.0, and 0.05% Tween 20.

The assay protocol included the following steps: 150 s baseline, 600 s association, 1500 s dissociation, 30 s denaturation in 1 M glycine-HCl (pH 2.0), and 150 s washing. Multiple rounds of measurements were performed, reusing the same sensor tip for increasing concentrations of PA_{Cter}. Kinetic parameters, including the association rate (kₒₙ), dissociation rate (k_{off}), and dissociation constant (K_{d}), were calculated using the Forte-Bio software. Results were further validated using custom R scripts based on the 1:1 binding model.

### Thermal Shift Assay (TSA)

TSA experiments were performed using a Stratagene MX 100 RT-PCR instrument in 96-well PCR plates with sealed covers. The temperature was increased from 25°C to 75°C in 1-minute intervals, and fluorescence measurements were recorded at each step. Sypro Orange (Thermo Fisher) was used as the hydrophobic marker at a 1000-fold dilution in a total volume of 20 µL per well. The final concentration of PA_{Cter} was 2 µM, diluted in a buffer containing 200 mM NaCl, 25 mM Tris pH 8.0, and 50 mM TCEP. Melting curves were generated by monitoring fluorescence changes, and the data were analyzed using custom R scripts. The melting temperature (Tₘ) was determined by taking the first derivative of the fluorescence signal with respect to temperature.

### Crystallisation and structure resolution

Peptide 4 was fused to the N-terminus of PA (A/California/07/2009, residues 254-716) using two-part overlap extension PCR (Bryksin et al. ([9]) from a synthetic DNA template (Thermo Fisher GeneStrings). The resulting construct was ligated into a vector between the Ncol and Xhol restriction sites, replacing the Wilson-Smith PA domain. Peptide 10 (P10) and Peptide 24 (P24) constructs were subsequently subcloned into the P4 construct via two-part ligation using Sacll and Ncol restriction sites, based on GeneString DNA templates. All constructs were transformed into E. coli Top10 chemically competent cells for amplification. Protein purification followed the same protocol as described for PA_{Cter}. Purified protein constructs were concentrated to 10 mg/mL and subjected to high-throughput crystallisation screening at the EMBL-HTX platform using CrystalDirect technology (Cipriani et al. ([10]). Matrix screens tested included Wizard Classic I and II (Rigaku Reagents), Grid Screen Salt HT (Hampton Research), and Morpheus (Molecular Dimensions). For conditions yielding crystals, manual crystallisation plates were set up with stepwise increments in pH and salt concentrations, using the hanging drop method with 1:1 and 1:2 reservoir-to-protein ratios. Crystals were manually harvested using various loops and cryoprotected by mixing for 30 seconds in a 20% glycerol/reservoir solution. For larger crystals, an intermediate step with 10% glycerol/reservoir solution was used before flash-freezing in liquid nitrogen. Crystals were loaded into Uni-Pucks for X-ray diffraction studies. X-ray diffraction data were collected at the European Synchrotron Radiation Facility (ESRF) on beamlines ID23-2 and ID30B (MASSIF-1), using the MXCuBe interface or automated data collection. Automated data processing was performed using the EDNA framework. The structure was solved via molecular replacement using the CCP4 suite, with the program MOLREP and the PA-PB1 structure (PDB: 2ZNL22) as the model. Refinement involved iterative cycles with REFMAC5 and model building in COOT. Final quality control was performed using SFcheck and MolProbity.

### Protein complementation assay

An Enhanced GFP plasmid (pEGFP-N1, GenBank U55762.1) was digested with Xhol and BamHI restriction enzymes and purified using the Macherey-Nagel NucleoSpin DNA kit. The digested plasmid was then ligated with partially complementary annealing primers in 1:3 or 1:6 molar ratios, generating a cassette with sticky ends complementary to the plasmid. The primer sequences were as follows:
Forward
   5'-CAGCGAAAGCAGGCAAACCATTTGA (SEQ ID NO: 79) and AATGCTATAAGCACAACTTTCCCTTATACTGGAGACC-3' (SEQ ID NO: 80), in which 45-nucleotide sequence encoding the peptide of interest is integrated; and
Reverse
   5'-TCAAATGGTTTGCCTGCTTTCGCTG (SEQ ID NO: 81) and GGTCTCCAGTATAAGGGAAAGTTGTGCTTATAGCATT-3' (SEQ ID NO: 82), in which 45-nucleotide sequence encoding the peptide of interest is integrated. The ligation was performed using T7 DNA ligase, and the product was transformed into E. coli Top 10 cells for amplification and DNA preparation. Cell viability was determined using the CellTiter-Glo Luminescent Viability Assay kit (Promega), following the manufacturer's protocol. Luminescent signals, proportional to the number of viable cells, were measured. Toxicity was quantified as relative light units (RLU), and cells showing less than 80% RLU compared to the control were deemed toxic. For the protein complementation assay, 50 ng of pCI plasmids encoding the PA and PB1 proteins from the A/WSN/33 (WSN) virus, fused to Gluc1 or Gluc2 (WSN-PA-Gluc1 and WSN-PB1-Gluc2, respectively), were used. Association of these proteins reconstituted an active Gaussia luciferase (Gluc) enzyme, serving as a competition assay for binding peptides. HEK-293T cells were transfected with 50 ng of p-PB1Nter-EGFP plasmid or 50 ng of empty pCI plasmid using polyethyleneimine (PEI-max, Polysciences Inc.; 3 µL of PEI at 1 mg/mL per 1 µg of DNA). As an alternative, pCI plasmids encoding PA and PB1 from the B/Memphis/13/2003 (B/Mem) virus, fused to Gluc1 or Gluc2 (B/Mem-PA-Gluc1 and B/Mem-PB1-Gluc2, respectively), were also used. After 24 hours, cells were lysed in Renilla lysis buffer (Promega) for 45 minutes at room temperature with gentle shaking. The luminescence signal was measured using a Centro XS LB960 microplate luminometer (Berthold Technologies), with a 10-second reading time following the injection of 50 µL Renilla luciferase reagent (Promega). Control experiments included the association of JUN-Gluc1 with FOS-Gluc2 in the presence of p-PB1-EGFP, measurement of Gaussia luciferase activity in the presence of p-PB1Nter-EGFP, and assessment of EGFP fluorescence using the Incucyte S3 system to monitor expression levels. All measurements were performed in technical triplicates 24 hours post-transfection. Disruption of the PA-PB1 interaction by peptides reduced the luciferase signal, which was expressed as normalized luminescence ratios (NLR) relative to the scrambled peptide control (pept Scr). The NLR was defined as the ratio of the signal to the sum of the signal from PA-Gluc1/Gluc2 and Gluc1/PB1-Gluc2. NLRs were expressed as percentages, with the scrambled peptide control set to 100%.

### Polymerase activity assay

HEK-293T cells were co-transfected with 25 ng each of pcDNA3.1-WSN-PB2, -PB1, and -PA plasmids, encoding the PB2, PB1, and PA proteins of the WSN viral polymerase complex. Additionally, 50 ng of pCI-WSN-NP, 10 ng of pPoll-Firefly, 5 ng of pTK-Renilla (Promega), and 50 ng of p-PB1-EGFP or empty pCI plasmid were co-transfected using polyethyleneimine (PEI, Polysciences Inc.). Viral polymerase activity was assessed by measuring Firefly luciferase expression driven by the viral RNA-dependent RNA polymerase (vRNA), while Renilla luciferase activity, driven by RNAP II, was used as an internal control for transfection efficiency. Both Firefly and Renilla luciferase activities were measured using the Dual-Glo Luciferase Assay System (Promega), following the manufacturer's instructions. All measurements were performed in technical triplicates 24 hours post-transfection (hpt). Firefly luciferase activity, reflecting active viral polymerase function, was normalized to Renilla luciferase activity to account for variations in transfection efficiency.

### Viral inhibition assay

mRNA LNPs encoding peptides 10, 24, the wild type and a scrambled version of the wild type in a random order were resuspended in PBS at a final concentration of 5 mM. A549 cells were seeded in 96-well plates (3x10⁴ cells/well). One day post-seeding, cells were washed with 100 µl of DMEM and infected with the WSN at a multiplication of infection (m.o.i.) of 10⁻⁴ PFU/cell for 1 h at 37°C, in 50 µl OptiMEM supplemented with 2% FCS. After removal of the virus, 100 µl of medium containing the indicated concentrations of peptides were added, in triplicates. Supernatants were collected from the triplicates and pooled. Virus particle production was assessed in a standard plaque assay. Cell viability in the presence of peptides was assessed in the same conditions, using the CellTiter-Glo Luminescent Viability Assay kit (Promega) as described above.

### Reference List

1. Behrendt R, White P, Offer J. Advances in Fmoc solid-phase peptide synthesis. J Pept Sci. 2016 Jan;22(1):4-27. doi: 10.1002/psc.2836. PMID: 26785684; PMCID: PMC4745034.
2. Rosano GL, Ceccarelli EA. Recombinant protein expression in Escherichia coli: advances and challenges. Front Microbiol. 2014 Apr 17;5:172. doi: 10.3389/fmicb.2014.00172. PMID: 24860555; PMCID: PMC4029002.
3. Obayashi, E. et al. The structural basis for an essential subunit interaction in influenza virus RNA polymerase. Nature 454, 1127-1131 (2008).
4. He, X. et al. Crystal structure of the polymerase PAC-PB1N complex from an avian influenza H5N1 virus. Nature 454, 1123-1126 (2008).
5. Moen, S. O. et al. Structural analysis of H1N1 and H7N9 influenza A virus PA in the absence of PB1. Sci. Rep. 4, 5944 (2015).
6. Kong, X.-D. et al.. Generation of a Large Peptide Phage Display Library by Self-Ligation of Whole-Plasmid PCR Product. ACS Chem. Biol. 15, 2907-2915 (2020).
7. Lee, C. M. Y., lorno, N., Sierro, F. & Christ, D. Selection of human antibody fragments by phage display. Nat. Protoc. 2, 3001-3008 (2007).
8. Zahnd, C., Sarkar, C. A. & Plückthun, A. Computational analysis of off-rate selection experiments to optimize affinity maturation by directed evolution. Protein Eng. Des. Sel. PEDS 23, 175-184 (2010).
9. Bryksin, A. V. & Matsumura, I. Overlap extension PCR cloning: a simple and reliable way to create recombinant plasmids. BioTechniques 48, 463-465 (2010).
10. Cipriani, F. et al. CrystalDirect: a new method for automated crystal harvesting based on laser-induced photoablation of thin films. Acta Crystallogr. D Biol. Crystallogr. 68, 1393-1399 (2012).
11. Sharma A, Kumar A, de la Torre BG, Albericio F. Liquid-Phase Peptide Synthesis (LPPS): A Third Wave for the Preparation of Peptides. Chem Rev. 2022 Aug 24;122(16):13516-13546. doi: 10.1021/acs.chemrev.2c00132. Epub 2022 Jul 11. PMID: 35816287.
12. Young E, Alper H. Synthetic biology: tools to design, build, and optimize cellular processes. J Biomed Biotechnol. 2010;2010:130781. doi: 10.1155/2010/130781. Epub 2010 Jan 27. PMID: 20150964; PMCID: PMC2817555.
13. Intarapat S, Stern CD. Chick stem cells: current progress and future prospects. Stem Cell Res. 2013 Nov;11(3):1378-92. doi: 10.1016/j.scr.2013.09.005. Epub 2013 Sep 24. PMID: 24103496; PMCID: PMC3989061.
14. Bahrami S, Amiri-Yekta A, Daneshipour A, Jazayeri SH, Mozdziak PE, Sanati MH, Gourabi H. Designing A Transgenic Chicken: Applying New Approaches toward A Promising Bioreactor. Cell J. 2020 Jul;22(2):133-139. doi: 10.22074/cellj.2020.6738. Epub 2019 Oct 14. PMID: 31721526; PMCID: PMC6874784.

## Claims

1. Influenza virus polymerase inhibitory peptide, comprising an amino acid sequence chosen among X₁DX₃NPX₆LLFLX₁₁X₁₂PX₁₄Q (SEQ ID NO: 1), OX₃NPX₆LLFLX₁₁X₁₂PX₁₄Q (SEQ ID NO: 2), X₁DX₃NPX₆LLFLX₁₁X₁₂PX₁₄ (SEQ ID NO: 3), and OX₃NPX₆LLFLX₁₁X₁₂PX₁₄ (SEQ ID NO: 4), wherein:
- X₁, when present, is chosen from V, I, L, M, Y, Q and W,
- X₃ is chosen from Y, F, I and M,
- X₆ is chosen from W and Y,
- X₁₁ is chosen from R, Q, L, K and V,
- X₁₂ is chosen from K, L, P, Q, V, F, and M, and
- X₁₄ is chosen from K, A, R Q, K, V, S and N.

2. Peptide according to claim 1, comprising a sequence chosen among X₁DX₃NPX₆LLFLX₁₁X₁₂PX₁₄Q (SEQ ID NO: 1) and X₁DX₃NPX₆LLFLX₁₁X₁₂PX₁₄ (SEQ ID NO: 3), wherein:
- X₁ chosen from V, I, L and W,
- X₃ is chosen from Y, F and M,
- X₆ is chosen from W and Y,
- X₁₁ is chosen from R, K and L,
- X₁₂ is chosen from K and Q, and
- X₁₄ is chosen from K and A.

3. Peptide according to claim 1 or 2, comprising a sequence chosen among X₁DX₃NPX₆LLFLX₁₁X₁₂PX₁₄Q (SEQ ID NO: 1) and X₁DX₃NPX₆LLFLX₁₁X₁₂PX₁₄ (SEQ ID NO: 3), wherein:
- X₁ is chosen from V and I,
- X₃ is chosen from Y and F,
- X₆ is chosen from W and Y,
- X₁₁ is chosen from R and K,
- X₁₂ is chosen from K and Q, and
- X₁₄ is K.

4. Peptide according to any one of the preceding claims, having an amino acid sequence selected among VDYNPWLLFLRKPKQ (SEQ ID NO: 6), IDYNPYLLFLKQPKQ (SEQ ID NO: 7), LDFNPWLLFLKLPAQ (SEQ ID NO: 8) and WDMNPWLLFLKLPRQ (SEQ ID NO: 9).

5. Peptide according to any one of the preceding claims, wherein one to four additional amino acids are added at the N-terminus, the C-terminus, or both.

6. Peptide according to any one of the preceding claims, which is conjugated to a labeling group, such as biotin, a fluorophore or a radioisotope.

7. Nucleic acid encoding a peptide as defined in any one of the preceding claims.

8. Nucleic acid according to claim 7, which is a messenger ribonucleic acid (mRNA).

9. Expression vector into which a nucleic acid according to claim 7 or 8 is inserted.

10. Cell transfected or modified genetically with an expression vector as defined in claim 9.

11. Vector for delivery of a nucleic acid as defined in claim 7 or 8, such as a Lipid Nanoparticle (LNP).

12. Medicament comprising a peptide as defined in any one of claims 1 to 6, or a nucleic acid as defined in claim 7 or 8, or a vector as defined in claim 11.

13. Peptide according to any one of claims 1 to 6, or nucleic acid as defined in claim 7 or 8, or vector as defined in claim 11, for use as a medicament.

14. Peptide according to any one of claims 1 to 6, or nucleic acid as defined in claim 7 or 8, or vector as defined in claim 11, for use in the treatment of influenza.

15. Method for preparing a peptide according to any one of claims 1 to 6, comprising the chemical synthesis of the peptide or the recombinant expression of the peptide.
